# EUROPEAN PATENT APPLICATION

(11) **EP 1 894 589 A1**
(43) Date of publication of application: **05.03.2008**
(21) Application number: 06742135.4
(22) Date of filing: 08.06.2006
(51) Int. Cl.: A61M 5/178, A61M 5/50

(54) **SELF-DESTRUCTED DISPOSABLE SYRINGE**

(30) Priority: 09.06.2005 CN 200520108852 U; 30.04.2006 CN 200610040147
(71) Applicant: Ying, Jishui, 325200 Ruian (CN)
(72) Inventor: CHEN, Qianquan, Ruian Zhejiang 325200 (CN); XU, Airong, Ruian Zhejiang 325200 (CN); ZHOU, Wanghan, Ruian Zhejiang 325200 (CN); YANG, Nanzhou, Ruian Zhejiang 325200 (CN)
(74) Representative: Gesthuysen, von Rohr & Eggert
(86) International application number: PCT/CN2006/001249
(87) International publication number: WO 2006/131074

(57) **Abstract**

A self-destructed disposable injection syringe is provided, the syringe includes a barrel (1) and an injecting plunger (2) which can slide within the barrel(1), a piston is provided on the end of the injecting plunger (2) within the barrel, a seal gasket (3) is provided on the piston (5), a heave (4) for destructing the seal gasket (3) is provided on the bottom of the barrel (1), a cavity (51) for the heave (4) entering is provided on the piston(5).

## Description

### FIELD OF THE INVENTION

The present invention relates to an injection syringe, especially a self-destructed disposable injection syringe.

### BACKGROUND OF THE INVENTION

At present, much more infectious diseases arose, and during the injection, the patients often use the injection syringe. In order to eliminate the cross infection caused by injection syringe, the disposable injection syringe is promoted. However, due to man-made factors, sometimes the injection syringe has the possibility of reusing and causes the cross infection. Thus, a self-destructed disposable injection syringe was invented, i.e., after used, the injection syringe is destructed immediately to ensure its disposable using purpose. This self-destructed disposable injection syringe usually consists of plunger, barrel and piston, it has a concave dangerous cross section on the plunger, after the injection completed, pull out the plunger, break the plunger from the dangerous cross section, thus destruct the injection syringe. This injection syringe shall be still manually destructed, not very easy to destroy. If the plunger is not broken due to the carelessness of nurses, it is still possible to reuse the injection syringe. Moreover, during injecting, the resistance of this injection syringe is relatively higher.

### SUMMARY OF THE INVENTION

The present invention provides a self-destructed disposable injection syringe, which is destroyed only after used on the first time

The present invention adopts the following technical solutions:

A self-destructed disposable injection syringe, consists of barrel and injecting plunger sliding within the barrel, a piston is provided on the end of the injecting plunger within the barrel, a seal gasket is provided on the piston, a projection is provided on the bottom of the barrel, a cavity for the projection entering is provided on the piston.

### Compared with the prior art, the present invention has the following advantages:

(1) During the application of the present invention, the injecting plunger may drive the piston on it to move toward the bottom of barrel, gradually closes to the bottom and butt against the projection at the barrel bottom when finish the injection cycle. The seal gasket is push up by projection and then deformed, so that part of the seal gasket broken and damaged. So, the present invention destructs itself when it is about to accomplish the first application, in order to avoid the possibility of reusing.
(2) The projection in the present invention adopts cutter for damaging the seal gasket, especially the projection is a cylinder with a slant top, the intersecting line between the cylinder and its top is a cutter, during the projection butts the projection on the bottom of the barrel, it may not only cause part of the seal gasket deformed and torn, but also cut the seal gasket by the cutter of projection thus the present invention can destructs itself more reliably when it is about to accomplish the first application.
(3) The hole on the piston cooperated with the cutter may cause the shearing fit with cutter as projection during the piston closes to bottom driven by the injecting plunge, exert the shear to seal gasket, thus the present invention can destructs itself more reliably and completely when it is about to accomplish the first application.
(4) The destructed area on the seal gasket is concave, which may reduce the thickness of the destructed area. It is favorable for destroying after used on the first time. The thickness of projection on the concave bottom is not uniform. It is favorable for destroying after used on the first time.
(5) In the present invention, the projection locates at the center of seal gasket made in sphere shape, which may ensure the split formed by the destruction of seal gasket matches with the shape of cutter. Due to the elasticity of seal gasket and split shape matching with the cutter, the split of seal gasket still remain the sealing with cutter before pulling back the injecting plunger and piston on it. Thus all injection fluid in barrel can be completely injected to fulfill the whole injection cycle.
(6) The tubular cutter for the present invention may shear the seal gasket. Thus the present invention can destructs itself more reliably and completely when it is about to accomplish the first application.. The cylinder matching with the tubular cutter may ensure the shearing of seal gasket. And ensure the split on seal gasket matches with the shape of cutter. Due to the elasticity of seal gasket and split shape matching with the cutter, the split of seal gasket still remain the sealing with cutter before pulling back the injecting plunger and piston on it. Thus all injection fluid in the barrel can be completely injected to fulfill the whole injection cycle.
(7) The head of cylinder extrudes the piston and inserts in the seal gasket. When the present invention is about to accomplish the first application, the seal gasket deformed and the cylinder head extruding the piston may ensure the seal gasket form a rigid heave, which directly acts on the bottom of barrel and also cause the barrel destructed. In the present invention, the external bottom of the barrel has a concave, which corresponding to the heave in the barrel, thus ensure the bottom of the barrel to be destroyed easy by the rigid heave.

### BRIEF DESCRIPTION OF THE DRAWINGS

Fig. 1 is the structure cutaway view of embodiment 1 described in the present invention.
Fig. 2 is the structure cutaway view of seal gasket described in the present invention.
Fig. 3 is the A-A cutaway view of embodiment 1 described in the present invention.
Fig. 4 is the magnified structural schematic diagram of piston on the end of the injecting plunger of embodiment 1 described in the present invention.
Fig. 5 is the structure cutaway view of embodiment 2 described in the present invention.
Fig. 6 is the B-B cutaway view of embodiment 2 described in the present invention.
Fig. 7 is the magnified structural schematic diagram of piston on the end of the injecting plunger of embodiment 2 described in the present invention.

### DETAILED DESCRIPTION OF PREFERRED EMBODIMENTS

Now referring to the attached figures, fulfill the detailed description for the execution of the present invention. The patent protection range of the present invention is, but not limited to the following embodiments.

### Embodiment 1

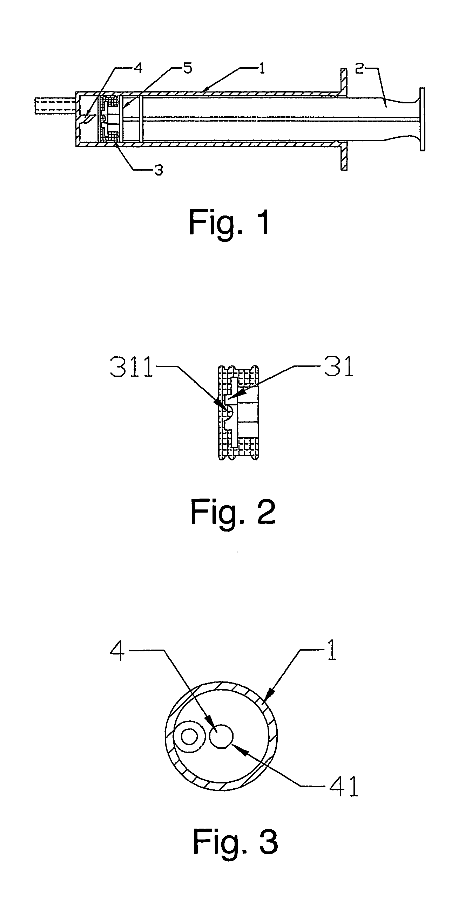

Referring to Fig. 1, Fig. 3 and Fig. 4, a self-destructed disposable injection syringe, consists of barrel 1 and injecting plunger 2, which is able to slide within barrel 1, the piston 5 is provided on the end of injecting plunger 2 in barrel 1, the seal gasket 3 is provided on the piston 5, a seal gasket 3 is provided on the piston 5, a heave 4 is provided on the bottom of the barrel 1, a cavity 51 for the heave 4 entering is provided on the piston 5.

The heave 4 is a cutter, so as to destroy the seal gasket 3 more easier after the injection syringe used on the first time. The cutter may have many options, such as sharp heave, blade, cylinder or other heave with cutting edge. In this embodiment, the heave 4 is a cylinder and its top is slant, the intersecting line between the cylinder and top surface is a cutting edge, the so-called slant cylinder top indicates that the top of cylinder is not perpendicular to the axis of cylinder, for example, the included angle between the top surface and axis of cylinder is 60°. In order to ensure the destruction of heave to seal gasket 3, in this embodiment, the piston 5 has a hole 52 which matches with the cutter. The hole 52 is integrated with the cavity 51, that is: the hole 52 may be deepened to accommodate the heave 4.

Referring to Fig. 2, the seal gasket 3 still has the following technical measures: the destructed area of seal gasket 3 is preset as concave 31, in order to ensure the destruction of the heave to the seal ring, the thickness of the seal ring at concave 31 area may be preset as different values, that is, its thickness is not uniform, in this embodiment, the bottom surface of concave 31 has the heave 311, which locates at the center of seal gasket 3 and made in sphere.

### Embodiment 2

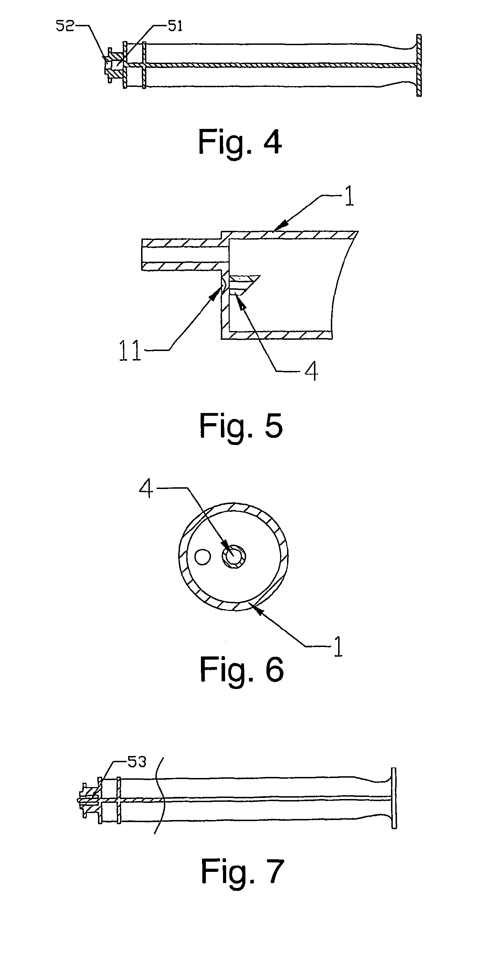

Referring to Fig. 5, Fig. 6 and Fig. 7, a self-destructed disposable injection syringe, consists of barrel 1 and injecting plunger 2, which is able to slide in barrel 1, the piston 5 is provided on the end of injecting plunger 2 in barrel 1, the seal gasket 3 is provided on the piston 5, the heave 4 for destroying the seal gasket 3 is provided on the bottom of barrel 1, the cavity 51 for the entering of heave 4 is provided on the piston 5, said heave 4 is a cutter, which has many options (referring to embodiment 1). In this embodiment, the heave 4 is a tubular cutter for destroying the seal gasket 3, the cavity 51 of piston 5 has cylinder 53 (Referring to Fig. 7) matched with tubular cutting edge, the external bottom of barrel 1 has a concave 11, which is opposite to heave 4 in barrel 1, ensure the concave 11 locate on the moving way of cylinder 53 driven by injecting plunger 2. The head of cylinder 53 extrudes beyond piston 5 and inserts into the seal gasket 3.

In the employ of the present invention, the injecting plunger may drive the piston on it toward the bottom of the barrel and gradually close to the bottom. And just before the injection completed, the seal gasket on the piston may act on the heave at the barrel bottom. Under the function of the heave, the seal gasket is pushed up by the heave and deformed. Partial seal gasket is broken and destroyed by the deformation and tore. Thus it realizes the self-destructed after the first time usage, Especially adopts the heave such as cylinder cutter, tubular cutter etc, just before the injection completed, the interaction between seal gasket and heave happened, and destroyed, utilize the elasticity of seal gasket, the split on seal gasket shall still keep the sealing state with cutting edge during pull back the injecting plunger and piston on it. Thus all injection fluid in barrel can be completely injected to realize a whole injection cycle.

## Claims

1. A self-destructed disposable injection syringe, the syringe comprising a barrel (1) and an injecting plunger (2) which can slide within the barrel (1), a piston (5) being provided on the end of the injecting plunger (2) within the barrel (1), a seal gasket (3) being provided on the piston (5), wherein a heave (4) for destructing the seal gasket (3) is provided on the bottom of the barrel (3) and a cavity (51) for the heave (4) entering is provided on the piston (5).

2. The self-destructed disposable injection syringe as in claim 1, wherein the heave (4) is cutter.

3. The self-destructed disposable injection syringe as in claim 2, wherein the heave (4) is a cylinder and its top surface is slant, the intersecting line between the cylinder and top surface is the cutting edge.

4. The self-destructed disposable injection syringe as in claim 2 or claim 3, wherein the hole (52) matched with cutter is provided on the piston (5).

5. The self-destructed disposable injection syringe as in claims 1 to 3, wherein the destroyed area on seal gasket (3) is concave (31).

6. The self-destructed disposable injection syringe as in claim 5, wherein the heave (311) is provided on the bottom of the concave (31).

7. The self-destructed disposable injection syringe as in claim 6, wherein the heave (311) locates at the center of seal gasket (3) and made in sphere.

8. The self-destructed disposable injection syringe as in claim 1, wherein the heave (4) is a tubular cutter for destroying the seal gasket (3).

9. The self-destructed disposable injection syringe as in claim 8, wherein the cylinder (53) matched with the tubular cutter is provided in the cavity (51) of piston (5).

10. The self-destructed disposable injection syringe as in claim 8, wherein the head of cylinder (53) extrudes beyond the piston (5).

11. The self-destructed disposable injection syringe as in claim 10, wherein the head of cylinder (53) inserts into the seal gasket (3).

12. The self-destructed disposable injection syringe as in claim 10 or claim 11, wherein the concave (11) is provided on the external bottom of barrel (1) and opposite to heave (4) in barrel(1).
